# FASCICULE DE BREVET EUROPEEN

(11) **EP 0 432 022 B1**
(45) Date de publication et mention de la délivrance du brevet: **23.10.1996**
(21) Numéro de dépôt: 90403406.3
(22) Date de dépôt: 30.11.1990
(51) Int. Cl.: C07K 1/02

(54) **Milieu réactionnel et solubilisant de peptides et procédé de synthèse de peptides utilisant ce milieu**
Medium zur Reaktion und Auflösung der Peptiden und Verfahren zur Herstellung von Peptiden durch Gebrauch davon
Medium for reaction and dissolution of peptides and process of peptide synthesis by making use of said medium

(30) Priorité: 04.12.1989 FR 8915957; 06.07.1990 FR 9008593
(43) Date de publication de la demande: 12.06.1991
(73) Titulaire: RHONE-POULENC CHIMIE, 92408 Courbevoie Cédex (FR)
(72) Inventeur: Galvez, Marie, F-69780 Toussieu (FR); Maurice, Marie-France, F-69300 Caluire (FR)
(74) Mandataire: Ricalens, François

(56) Documents cités:
- GB-A- 1 182 450
- CHEMICAL ABSTRACTS, vol. 98, no. 13, 28 mars 1983, page 642, résumé no. 107742u, Columbus, Ohio, US; M. BODANSZKY et al.: "Coupling in the absence of tertiary amines", & INT. J. PEPT. PROTEIN. RES. 1982, 20(4), 387-95
- BULLETIN OF THE CHEMICAL SOCIETY OF JAPAN, vol. 44, no. 4, avril 1971, pages 1108-1111; M. UEKI et al.: "Peptide synthesis by oxidation-reduction condensation. I. Use of NPS-peptides as amino component"

## Description

La présente invention a pour objet un milieu réactionnel et solubilisant pour la synthèse de peptides. Elle a aussi pour objet un procédé de synthèse de peptides dans ce même milieu.

Il existe un grand nombre de méthodes de synthèse de peptides en phase liquide. Elles ont toutes des points communs de base qui consistent:
- à protéger éventuellement la fonction de la chaîne latérale d'un amino-acide par un groupe protecteur clivable en fin de synthèse du peptide;
- à protéger la fonction amine (N α) dudit amino-acide par un groupement protecteur clivable après la condensation de l'amino-acide;
- à activer la fonction acide carboxylique dudit amino-acide protégé;
- puis à le condenser avec un acide aminé ou un peptide dont la fonction C terminale est protégée et dont la fonction amine est libre.
- le peptide est obtenu par déprotection totale des groupes protecteurs après la condensation de tous les amino-acides.

La réaction de condensation peut être réalisé aussi bien en phase liquide homogène qu'en phase hétérogène (par exemple synthèse de Merrifield).

En général, la synthèse de peptides nécessite la protection de la fonction carboxylique de l'acide aminé C terminal sous forme d'esters.

Dans le cas des synthèses peptidiques en phase homogène, l'ester sera choisi parmi:
- les esters méthyliques, benzyliques, tertiobutyliques,
- les esters de polymères solubles dans les solvants organiques:
   . par exemple les esters de polyéthers de MUTTER M. et Al Justus Liebigs Annalen der Chemie 1975 pp. 901. 915.
- ou mieux parmi les esters revendiqués dans le Brevet R.P. déposé par la demanderesse (n° FR 89 06700 sur les esters GPC).

Dans le cas des synthèses peptidiques en phase hétérogène,
- l'ester pourra être notamment un ester de polymères non solubles dans les solvants organiques.

On peut citer de manière non exhaustive les polymères suivants:
* le copolymère styrène divinylbenzène introduit par R.B. MERRIFIELD (J.A.C.S. 1963. 85. p. 2149, 2154);
* le polydiméthyl acrylamide ―co-Boc-β-Ala-N′-acroylyl-hexaméthylénédiamine de E. ATHERTON et R.C. SHEPPARD (J.A.C.S. 97 1975 p. 6584.6585);
* le polystyrène greffé sur Kel-F (TREGEAR and AI 1966 U.S. Patent: 3 700 609 and Chem. Abot. 71 p 508241 (1969));
* la cellulose.

La mise en oeuvre des synthèses peptidiques en phase hétérogène peut se faire soit en réacteur agité, soit en réacteur colonne, soit par toute autre technique (par exemple utilisation de membrane)

Le rendement et la pureté du produit final dépendent beaucoup du rendement de chaque étape, d'une part en raison de l'accroissement géométrique des pertes, et d'autre part en raison des problèmes de séparation du produit désiré d'avec les sous produits. Ce problème s'amplifie au cours de la progression de la synthèse lorsque le nombre d'acides aminés augmente.

En outre, dans le cas spécifique où on réalise la condensation en milieu liquide homogène, toutes ces méthodes présentent un même inconvénient: leur productivité volumique est faible en raison de la mauvaise solubilité des acides aminés ou des peptides protégés intermédiaires.
Cet inconvénient est signalé par de nombreux auteurs tels que:
. M. NARITA, K. ISHIKAWA, J.Y. CHEN et Y. KIM, Int. J.Peptide Protein Res., 24, 580 (1984).
. E. GROSS and J. MEIENHOFER, The Peptides; Analysis, Synthesis, Biology, Academic Press, 1, 45, (1979).
. M. MUTTER et E. BAYER, The Peptides; Analysis, Synthesis, Biology, Academic Press, 2, 288 (1980).
. FUHRHOP and PENZCIN, Organic Synthesis, Verlag Chemie, 4.1.2. Peptides, 219 (1984).

a) La productivité par unité de volume est en particulier très faible lorsque l'on utilise des solvants non miscibles à l'eau tels que le chloroforme ou l'acétate d'éthyle. A titre d'exemple le nitro 4 benzyl ester de la N-(Nitro-2 phénylsulfényl)-α benzyl-phénylalanyl-N ω nitro-L-arginine est synthétisé à raison de 0,016 mole par litre au sein du chloroforme: E. Wünsch, Methoden der Organischen Chemie, XV-2, Synthese von Peptiden, Georg Thieme Verlag, 108 (1974).

Pour sa part, l'ester méthylique de la benzyloxycarbonyl-L-prolyl-L-tyrosine a été préparé à la concentration de 0,050 mole par litre au sein de l'acétate d'éthyle:

M. BODANSKY, A. BODANSKY, The Practice of Peptide Synthesis, Springer-Verlag, 140, 1984.

Les solvants miscibles à l'eau présentent des inconvénients spécifiques et ne sont guère plus avantageux.

En effet, s'ils permettent parfois de faire la synthèse des peptides en milieu plus concentré, ils en interdisent la purification directe par lavage à l'eau, lequel est nécessaire pour éliminer les réactifs introduits en excès et les produits de la réaction de couplage.

De ce fait, les solvants miscibles à l'eau sont habituellement distillés puis remplacés par des solvants non miscibles à l'eau, afin de procéder aux lavages. On revient donc au problème précédent car l'étape de purification nécessite des volumes de solvant très importants, cependant que le traitement de la masse réactionnelle a été rendu plus complexe par l'introduction des opérations supplémentaires de distillation et de redissolution.

Ainsi, le dipeptide Z-Cys(S-BZl)-Tyr OEt préparé dans le THF (2 mole/litre) a été tranféré dans l'acétate d'éthyle (0,1 mole/litre) avant d'être lavé à l'eau.
M. BODANSKY, The Practice of Peptide Synthesis, Springer Verlag, 129, 1984.

Le dipeptide protégé Z-Lys(Z)-Gly-OEt synthétisé dans l'acétonitrile (0,13 mole/litre) a été purifié après remplacement du solvant initial par de l'acétate d'éthyle (0,05 mole/litre, Ibid 150, 1984) tandis que Z-Ala-Tyr OCH₃ a été préparé dans le diméthylformamide (0,3 mole/litre) avant d'être purifié, par lavage à l'eau, en solution dans l'acétate d'éthyle (0,1 mole/litre) (Ibid 148, 1984).

L'emploi de ces solvants miscibles à l'eau, qui en général sont polaires, peut amener des inconvénients supplémentaires tant sur le plan de l'hygiène industrielle (DMSO, HMPT), que sur le plan de la sélectivité chimique. D'une manière générale, les solvants polaires favorisent en effet la racémisation de l'acide aminé activé N-protégé, selon la mise au point de D.S. KEMP, Peptides, Analysis, Synthesis, Biology; 1, 354-355, 1979.

Ainsi l'obtention de solutions concentrées d'intermédiaires peptidiques dans des solvants organiques non miscibles à l'eau et compatibles avec les normes actuelles de fabrication (Good Manufacturing practices) reste un problème non résolu.

C'est pourquoi un des buts de la présente invention est de fournir un milieu réactionnel qui augmente le rendement des condensations peptidiques.
Un autre but de la présente invention est de trouver un milieu réactionnel qui permette de faciliter les synthèses et les purifications des peptides, notamment les peptides de 2 à 50 acides aminés en général, et en particulier de 3 à 20 acides aminés, ou amino-acides.

Un autre but enfin est de fournir un milieu réactionnel permettant une synthèse peptidique en milieu homogène à des concentrations élevées (par exemple au moins égal à 0,1 en général à 0,2 M.

Ces buts et d'autres qui apparaitront par la suite, sont atteints par un milieu réactionnel et solubilisant pour la synthèse et/ou la purification peptidique qui comporte un diluant A choisi dans le groupe des solvants non miscibles à l'eau et un phénol B, le rapport massique entre ledit phénol B et ledit diluant A étant compris entre 1/200 et 1, de préférence compris entre 1/20 et 1/2 et comportant au moins un acide aminé ou au moins un peptide à une concentration au moins égale à 10⁻² M. Ainsi, la quantité de phénol B est de préférence d'au moins 1/200 du diluant A.
Il n'existe pas de limite supérieure stricte. Ainsi, l'utilisation de phénol B (produit pur ou mélange) sans diluant A, fait partie de l'invention dans la mesure où les conditions suivantes sont remplies :
- Le point de fusion du phénol B est au plus égal à 50 °C (dans la présente description les zéros ne sont pas dès chiffres significatifs sauf si cela est spécifié autrement) ;
- Le phénol B peut être séparé d'avec le produit réactionnel par distillation éventuellement ou pression réduite ;
- Le phénol B n'est pas miscible à l'eau en toute proportion ;
- Le phénol B ne forme pas d'émulsion stable susceptible de gêner l'élimination des co-produits par lavage à l'eau.
Les halogéno phénols avantageusement di-, et de préférence mono chloro, ainsi que les alcoyl phénols légers répondent par exemple à ces contraintes.

S'ils peuvent être utilisés seuls, les phénols répondant aux dites contraintes constituent également des sous-classes particulièrement intéressantes des phénols utilisables en mélange avec les diluants A.

Il est toutefois préférable qu'il y ait un diluant A et un phénol B.
Avantageusement, le rapport massique entre ledit phénol B et ledit diluant A est compris entre 1/200 et 1, de préférence entre 1/20 et 1/2.
Les valeurs ci-dessus exprimées en rapport massique conviennent bien pour le phénol lui-même et pour les phénols dont la masse moléculaire n'est pas d'un ordre de grandeur différent de celui du phénol proprement dit (C₆ H₅ OH).
Pour les phénols de poids moléculaires élevés, la concentration en mole par litre est au moins égal à 5 10⁻², avantageusement entre 0,1 et 2 de préférence entre 0,5 et 1,5 M.

Lorsque les diluants A et les phénols B ne sont pas miscibles en toute proportion, la limite supérieure de la teneur en phénol(s) est la plus basse des deux limites: celle mentionnée ci-dessus et la limite de solubilité.
Par l'expression phénol B, il faut comprendre des phénols ou des mélanges de phénols.
Les diluants A sont des solvants organiques suffisamment polaires pour dissoudre au moins 1% de préférence au moins 2% en masse du phénol (C₆ H₅ OH) et sont suffisamment hydrophobe pour n'être pas miscible à l'eau en toute proportion.

Il est préférable que l'eau ne puisse dissoudre qu'au plus 10% de diluant A avantageusement au plus 1% en masse et ce même en présence du phénol B en tant que tiers solvant.

Les diluants A peuvent être des mélanges, y compris des fractions pétrolières. Naturellement, dans les conditions opératoires les diluants A doivent être inertes vis-à-vis des phénols et des réactifs de synthèse peptidique utilisés.
Les familles préférées de diluants sont choisis dans le groupe constitué par les dérivés aromatiques, les ethers, les esters et les solvants halogénés.

A titre de paradigmes de membres de ces familles on peut citer comme dérivés aliphatiques halogénés le dichlorométhane, le dichloro-1,2-éthane, le trichloro-1,1,1- éthane, comme dérivés aromatiques le toluène et comme dérivés aromatiques halogénés le chlorobenzène, comme esters l'acétate d'éthyle et l'acétate d'isopropyle, comme éthers, l'oxyde de tertiobutyle et de méthyle ainsi que l'anisole.
Pour des raisons d'économie industrielle, il est préférable que le diluant A soit distillable sous pression atmosphérique ou sous vide primaire ou secondaire.
En général le phénol B est choisi dans le groupe des composés répondant à la formule suivante I :

(R₁)n - Ar - O - H (I)

dans laquelle :
- Ar représente un radical aromatique monocyclique, polycyclique, hétérocyclique ou non ;
- Les substituants R₁, semblables ou différents, représentent :
   . un motif halogène, de préférence fluor, chlore, brome
   . un groupe -Z-R₂, où Z peut être
      - une simple liaison ;
      - un atome d'oxygène ;
où R₂ représente un atome d'hydrogène, un radical alcoyle ou aryle éventuellement hydroxylés ou mono ou poly halogénés d'au plus 8 atomes de carbone,
- où n représente le nombre de substituants et est égal à 0 ou à un entier au plus égal au nombre de positions substituables sur les noyaux aromatiques ; et de leurs mélanges.

Les groupes alcoyles (tel que défini dans le dictionnaire de la Chimie Duval Presses Scientifiques Internationales PARIS VIe 1959) peuvent notamment être des restes aliphatiques linéaires ou ramifiés d'au plus 6 atomes de carbone ou des restes arylaliphatiques.

Le nombre de positions substituables est déterminable aisément à l'aide de règles simples connues de l'homme de métier.

Ainsi par exemple :
- quand Ar =: phényle n ≤ 5
- Ar =: pyridyle ≤ 4
- Ar =: naphtyle ≤ 7
- Ar =: quinolyle ≤ 6

Avantageusement le phénol B a un nombre d'atomes de carbone d'au plus 30 atomes de carbone, de préférence au plus 20 atomes de carbone.

Il est souhaitable que les positions vicinales de la fonction phénol soient non substituées ou occupées par des groupes non encombrants.
Sont considérés comme encombrants les radicaux reliés aux dites positions vicinales par un carbone tertiaire voire secondaire.
Les composés monocyclique sont ceux qui donnent le meilleur compromis efficacité-coût ; on préfère ceux à 6 chaînons (noyau pyridinyle ou phényle).

Il est également souhaitable que Z-R₂ ne puissent signifier plus de trois fois de préférence plus de 2 fois un groupe hydroxyle.

Avantageusement, dans la formule I, les radicaux R₁ sont choisis parmi le groupe constitué par :
- les radicaux méthyle, éthyle, propyles, butyles,
- les radicaux trifluorométhyle et pentafluoro éthyle,
- les radicaux méthoxyle, ethoxyle, propyloxyle, butyloxyle,
- phényle, hydroxy phényle, et -Ar-OH,
- les radicaux phényloxyle, hydroxy phényloxyle,
- atomes de fluor, de chlore et de brome.

Pour ne pas trop alourdir la molécule de phénol, il est souhaitable que dans la formule I n soit au plus égal à 5, de préférence à 3.
Parmi les phénols donnant les meilleurs résultats, il convient de citer :
- les hydroxy pyridines éventuellement monosubstituées,
- les hydroxy quinoleines éventuellement monosubstituées,
- les mono halogéno phénols (de préférence mono chloro),
- les poly halogéno phénols, (de préférence polyfluoro),
- les phénols mono ou disubstitués par des radicaux alkyles de C₁ à C₄, alcoxyles de C₁ à C₄, des perfluoroalkyle de C₁ à C₂ et le 2, 2, 2 trifluoro éthyle,
- les diphénols,
- le phénol au sens étroit,
- les naphtols éventuellement mono- ou di-substitués.

Les milieux selon la présente invention permettent de dissoudre un acide aminé éventuellement protégé, et ce à une concentration au moins égale à 0,05 M.
L'acide aminé peut être C ou N protégé; dans ce dernier cas, le groupement acide peut être activé sur la fonction acide, par exemple, le cas échéant, par le(s) fonctions acide(s) latérale(s) (cas de diacides, notamment aspartique). Les acides aminés peuvent être naturels ou synthétiques.
Le milieu selon la présente invention permet également de dissoudre un peptide protégé.
Le milieu selon la présente invention permet également de dissoudre des peptides non protégés (HCl, Pro-Gly-NH₂; HCl, His-Trp; 2HCl, Arg-Pro) et faire la synthèse.
Du fait que le milieu selon la présent invention solubilise à la fois acides aminés, peptides et réactifs de blocage ou d'activation, il permet de réaliser "in situ" les blocages, voire l'activation des acides aminés.
Ainsi le milieu selon la présente invention peut jouer un rôle de réactif quand il comporte en vue d'une introduction successive ou simultanée, outre le phénol B et le diluant A, (en général pour être plus pratique le milieu selon l'invention tout prêt) des peptide(s) (y compris les acides aminés) et/ou réactifs de blocage et/ou d'activation. Une phase aqueuse non miscible avec le milieu réactionnel selon l'invention peut être un composant supplémentaire du réactif.
Le peptide, y compris l'acide aminé élémentaire, peut être C ou N protégé; dans ce dernier cas, le groupement acide peut être activé sur la fonction acide.
Bien sûr, les fonctions des acides aminés, autres que les fonctions acide ou amine visées par la synthèse, peuvent être également protégées si nécessaires selon les techniques usuelles en la matière.

Le milieu de la présente invention permet d'augmenter notablement la solubilité des peptides en phase organique.

Il permet, en général, de changer l'ordre de grandeur de la solubilité dans le sens favorable, en prenant pour base la solubilité du peptide dans le diluant A organique en l'absence d'additif phénolique.

Le milieu permet de réaliser les différentes étapes de la synthèse de peptides dans des conditions permettant un bon rendement volumique. Il augmente en particulier la productivité des étapes de condensation.

Parmi les nombreux avantages du milieu selon la présente invention, il convient de souligner que les condensations d'un acide aminé ou d'un peptide N protégé et C activé avec un acide aminé ou un peptide non C protégé, sont très faiblement facilitées et accélérées lorsque la réaction a lieu dans ledit milieu. Ceci présente un avantage très appréciable pour la synthèse des oligo peptides (jusqu'à environ vingt chaînons, voire cinquante). C'est une des raisons pour lesquelles il convient de faire mention particulière des milieux selon l'invention jouant le rôle de réactif et qui contiennent comme composants en vue d'une introduction successive ou simultanée:
a) un diluant A choisi dans le groupe des solvants non miscibles à l'eau;
b) un phénol B;
c) un oligo peptide de 1 à 50, avantageusement de 1 à 20 acides aminés dont ni le N ni le C terminal n'est protégé.
La quantité de phénol B est de préférence d'au moins 1/200 du diluant A.

Il n'existe pas de limite supérieure stricte. Avantageusement, le rapport massique entre ledit phénol B et ledit diluant A est compris entre 1/200 et 1, de préférence entre 1/20 et 1/2.
Les valeurs ci-dessus exprimées en rapport massique conviennent bien pour le phénol lui-même et pour les phénols dont la masse moléculaire n'est pas d'un ordre de grandeur différent de celui du phénol proprement dit (C₆H₅OH).
Pour les phénols de poids moléculaires élevés, la concentration en mole par litre est au moins égale à 5-10⁻², avantageusement entre 0,1 et 2 de préférence entre 0,5 et 1,5 M.

Lorsque les diluants A et les phénols B ne sont pas miscibles en toute proportion, la limite supérieure de la teneur en phénol(s) est la plus basse des deux limites: celle mentionnée ci-dessus et la limite de solubilité.
La teneur en oligo-peptide, lequel dans la présente acception comprend le cas de l'acide amino non condensé, est avantageusement au moins égal à 10⁻² M de préférence 0,1 M. La concentration supérieure correspond à la limite de solubilité étant bien entendu que l'on peut utiliser une suspension d'oligo peptide si sa solubilité dans le milieu est jugée insuffisante par l'homme de métier.

La présente invention vise également un procédé de synthèse de peptides éventuellement protégés en milieu liquide, où l'on part d'un peptide ou d'un amino acide initial, protégé éventuellement sur sa fonction acide et l'on ajoute un aminoacide activé sur la fonction acide et protégé sur la fonction amine, dans lequel les réactifs sont dissous dans le milieu selon l'invention.
De la même manière, la présente invention vise aussi un procédé de synthèse de peptides éventuellement protégés en milieu liquide, où l'on part d'un amino acide ou peptide initial, solubilisé selon l'invention et l'on condense un autre peptide ayant sa fonction acide C terminale activée et sa fonction amine N-terminale protégée, dans lequel les réactifs sont dissous dans un milieu selon l'invention.

Par exemple la fonction amine est protégée par un groupe carbamate ou par réaction avec un composé bétadicarbonylé.

Avantageusement, la fonction acide des réactifs N-protégés est activée par un chlorure d'acide organique ou minéral, par un chloroformiate d'alkyle, par un carbodiimide ou par un ester activé ou par leur carbonyl di imidazole.

Après condensation d'un amino-acide ou d'un peptide -N protégé avec le peptide ou l'amino acide initial, la fonction amine -N-terminale est libérée par toute méthode, et, avantageusement par hydrogénolyse ou par solvolyse acide ou basique ou par photolyse.

L'amino-acide ou le peptide C-terminal protégé (de préférence par esterification ou par amidation) initial est, en général introduit dans le milieu réactionnel, puis l'acide aminé ou le peptide activé sur sa fonction acide et protégé sur sa fonction amine est ajouté, l'ordre d'addition des réactifs étant indifférent, après réaction les co-produits et les excès de réactifs sont éliminés de la phase organique par lavage avec des solutions aqueuses acides, basiques ou neutres, puis la fonction amine est libérée, et enfin un nouvel acide aminé ou un nouveau peptide activé sur sa fonction acide et protégé sur sa fonction amine, est introduit.

De manière surprenante, le milieu potientialise la réactivité des peptides avec les acides aminés et améliore significativement la cinétique de la condensation.

Un avantage important du procédé est qu'il permet d'éliminer de la phase organique concentrée les réactifs en excès et les co-produits de condensation par simple lavage à l'eau, facilité par l'effet antiémulsionnant des phénols, effet constaté au cours de l'étude qui a mené à la présente invention. Les réactifs en excès sont par exemple des amino acides ou peptides activés et N-protégés, soit des amino-acides ou peptides C-protégés, soit encore des catalyseurs servant à accélerer le couplage tels que l'hydroxy benzotriazole, l'imidazole, le N-hydroxysuccinimide, etc.....

Le milieu selon la présente invention permet également d'augmenter la productivité des étapes de clivage des groupes protecteurs. Il permet en outre, de réaliser aussi bien le clivage des groupes acidosensibles, comme par exemple le t-butyloxycarbonyl, au moyen de réactifs courants tels que l'acide trifluoracétique ou l'acide chlorhydrique, que le clivage des groupes hydrogénolysables, comme par exemple le benzyloxycarbonyl, les éthers et esters benzyliques ou le groupe nitro protégeant la fonction guanidino de la chaîne latérale de l'arginine.

Ledit milieu permet la coupure des groupes basolabiles tels que le sulfofluorényl-méthyl- oxycarbonyle par des réactifs courants tels la diéthylamine ou la pipéridine. Il permet aussi la coupure des groupes protecteurs photolysables. Il permet également la coupure des groupes protecteurs par réduction électrochimique.

Un avantage important du procédé est, en phase homogène, qu'il permette l'élimination des catalyseurs des étapes de clivage par des opérations simples: les catalyseurs acides ou basiques, peuvent être éliminés par lavage aqueux tandis que les catalyseurs d'hydrogénolyse tels que le palladium absorbé sur charbon sont éliminables par filtration; le peptide ou intermédiaire peptidique restant en solution organique concentrée.

Un autre avantage du procédé de solubilisation est qu'il est compatible avec toutes les opérations nécessaires à la synthèse des peptides et qu'il permet un enchaînement direct entre les différentes étapes de condensation, de clivage de groupes protecteurs et de purification par lavage à l'eau sans changement de solvant, et éventuellement sans isolement du peptide. Il permet donc la synthèse répétitive, ou plutôt itérative, de peptides.

Du fait de la répétitivité de la synthèse, le procédé de la présente invention peut être avantageusement mis en oeuvre sous une forme automatisée.

Le procédé est particulièrement intéressant pour la synthèse de peptides peu solubles dans les solvants organiques tels que les peptides renfermant des résidus glycyle, arginyle, glutaminyle, asparaginyle, serinyle, thréonyle et prolinyle;
En ce qui concerne les paramètres non visés par la présente description, le procédé selon la présente invention est réalisé dans les diverses conditions usuelles.

Les peptides issus de la synthèse peuvent être utilisés éventuellement pour la synthèse de médicaments, vaccins, produits agroalimentaires ou phytosanitaires.
La présente invention est illustrée par des exemples non limitatifs suivants.

### Protocole des essais de solubilisation

Les solubilités de l'intermédiaire peptidique ont été déterminées comme suit, à la température de 25 °C.

Une quantité pesée exactement de l'intermédiaire peptidique est placée dans un tube en pyrex. Le solvant ou mélange de solvants à l'essai est alors introduit à l'aide d'une pipette de précision de façon à obtenir un rapport masse de peptide/volume de solvant égal à 8g/100cm³.
Le tube fermé par un bouchon à vis est alors placé sur un agitateur de marque HEIDOLPH, modèle TOP-MIX, vibrant à sa vitesse maximale durant 60 secondes.
Dans le cas où le solide est entièrement dissous, la solubilité est notée > 8g/100cm³, sinon le volume de solvant est doublé et le processus recommencé.

A l'issue de 4 dilutions successives sans dissolution totale, les mesures sont reprises sur une quantité plus faible de solide.
Lorsque la solubilité dans un solvant donné a été trouvée inférieure à 0,05g/100cm³, il a été en outre vérifié que le chauffage à 40°C et une agitation supplémentaire de 4 minutes ne changent pas le résultat.
Remarques : . la composition des mélanges solvant/additif est exprimée pondérablement.

### SOLUBILISATION DE DIFFERENTS PEPTIDES

### EXEMPLE 1 : Chlorhydrate de l'ester méthylique de la L-serinyl-L-tyrosine. (HCl, Ser-Tyr-OCH₃)

### RESULTATS

Solubilité dans le dichlorométhane : s < 0,05g/100cm³

Solubilité dans le mélange dichlorométhane/phénol; 4/1 : 8>s>4g/100cm³

### Exemple 2 : Trifluoracétate de l'ester méthylique de la L-tryptophanyl-L-serinyl-L-tyrosine (CF₃ COOH, Trp-Ser-Tyr-OCH₃):

Les solubilités mesurées comme dans l'exemple 1 sont les suivantes :
. dans le dichlorométhane : s<0,05g/100cm³.
. dans le mélange dichlorométhane/phénol; 4/1 : 8>s>4g/100cm³.

### Exemple 3 : Ester méthylique de la L-tryptophanyl-L-serinyl-L-tyrosine (Trp-Ser-Tyr-OCH₃) :

Solubilités mesurées comme dans l'exemple 1 :
. dans le dichlorométhane : s < 0,05g/100cm³
. dans le mélange dichlorométhane/phénol ; 4/1 : s > 8g/100cm³
. dichlorométhane/t-butyl-4-phénol ; 4/1 : s > 8g/100cm³
. dichlorométhane/diméthoxy-2,6-phénol ; 4/1 : s > 8g/100cm³
. dichlorométhane/diméthyl-2,6-phénol ; 4/1 : s > 8g/100cm³
. dichlorométhane/dichloro-2,6-phénol ; 4/1 : s > 8g/100cm³
. dichlorométhane/hydroxy-2-pyridine ; 5/1 : s > 8g/100cm³
. dichlorométhane/méthoxy-2 phénol ; 4/1 : s > 169/100 cm³
. dichlorométhane/trifluorométhyl-3-phénol ; 4/1 : s > 8g/100cm³
. dichlorométhane/pentafluoro-2,3,4,5,6 phénol ; 4/1 : s > 8g/100cm³
. acétate d'éthyle : s < 0,05g/100cm³
. acétate d'éthyle/phénol ; 9/1 : s = 8g:100cm³

### Exemple 4 : Chlorhydrate du -L-histidinyl-L-tryptophane (HCl, His,Trp) :

Solubilités mesurées comme dans l'exemple 1 :
. dichlorométhane : s < 0,05g/100cm³
. dichlorométhane/phénol ; 4/1 : s = 2 g/100cm³

### Exemple 5 : N-Benzyloxycarbonyl-L-pyroglutanyl-L-histidine (Z-p Glu-His)

Solubilités mesurées comme dans l'exemple 1 :
. dichlorométhane : s < 0,05g/100cm³
. dichlorométhane/phénol ; 4/1 : 8 > s > 4g/100cm³
. dichlorométhane/méthoxy-4-phénol ; 4/1 : 2 > s > 1g/100cm³
. dichlorométhane/hydroxy-2-pyridine ; 5/1 : s = 0,3g/100cm³
. dichlorométhane/trifluorométhyl-3-phénol ; 4/1 : 8 > s > 4g/100cm³
. chloro-2-phénol : 8 > s > 4g/100cm³
. pentafluorophénol/dichlorométhane ; 1/4 : 8 > s > 4g/100cm³

### Exemple 6 : Dichlorhydrate de la -L-arginyl-L-proline ( 2 HCl, Arg-Pro)

Solubilités mesurées comme dans l'exemple 1 :
. dichlorométhane : s < 0,05g/100cm³
. dichlorométhane/phénol ; 4/1 : s = 2 g/100cm³
. dichlorométhane/chloro-2-phénol ; 1/1 : 2 > s > 1g/100cm³

### Exemple 7 : Chlorhydrate du L-leucyl-L-arginyl-L-prolyl-glycinamide (HCl, Leu-Arg-Pro-Gly-NH₂)

Solubilités mesurées comme dans l'exemple 1 :
. dichlorométhane : s < 0,05g/100cm³
. dichlorométhane/méthoxy-4-phénol 4/1 : s = 4g/100cm³
. dichlorométhane/phénol ; 4/1 : s = 4g/100cm³
. dichlorométhane/trifluorométhyl-3-phénol ; 4/1 : s = 4g/100cm³
. dichlorométhane/chloro-2-phénol; 2/1 : 2 > s > 1g/100cm³
. dichlorométhane/pentafluoro-2,3,4,5,6-phénol ; 4/1 : 4 > s > 2g/100cm³
. chloro-2-phénol : 4 > s > 2g/100cm³
. dichlorométhane/tertiobutyl-4-phénol; 4/1 : 1 > s > 0,5g/100cm³
. dichlorométhane/hydroxy-2-pyridine ; 5/1 : s = 1 g/100cm³

A titre comparatif,le diméthylformamide, additif qui ne fait pas partie de l'invention, a été essayé :
. dichlorométhane/diméthylformamide ; 4/1 : s < 0,5g/100cm³

### Exemple 8 : Chlorhydrate du L-prolyl-glycinamide (HCl, Pro-Gly-NH₂).

Solubilités mesurées comme dans l'exemple 1 :
. dichlorométhane : s < 0,05g/100cm³
. dichlorométhane/phénol ; 4/1 : s > 16g/100cm³
. dichlorométhane/méthoxy-4-phénol ; 4/1 : 8 > s > 4g/100cm³
. dichlorométhane/chloro-2-phénol ; 2/1 : 2 > s > 1g/100cm³
. dichlorométhane/hydroxy-2-pyridine ; 5/1 : 4 > s > 2g/100cm³

A titre comparatif le diméthyl formamide, additif qui ne fait pas partie de l'invention a été essayé :
. dichlorométhane/diméthylformamide; 4/1 : s < 0,5g/100cm³

### Exemple 9 : N-ε-Trifluoracétyl-L-Lysyl-L-proline (Lys (TFA)- Pro)

Solubilités mesurées comme dans l'exemple 1 :
. dichlorométhane : s < 0,05 g/100cm³
. acétate d'éthyle : s < 0,05 g/100cm³
. dichlorométhane/phénol ; 4/1 : s > 20 g/100cm³
. dichlorométhane/tertiobutyl-4-phénol ; 4/1 : s > 8 g/100cm³
. dichlorométhane/diméthoxy-2,6-phénol ; 4/1 : s > 8 g/100cm³
. dichlorométhane/diméthyl-2,6-phénol ; 4/1 : s = 8 g/100cm³
. dichlorométhane/hydroxy-2-pyridine ; 5/1 : s > 8 g/100cm³
. dichlorométhane/dichloro-2,6-phénol ; 4/1 : s > 8 g/100cm³
. dichlorométhane/méthoxy-2-phénol ; 4/1 : s > 8 g/100cm³
. dichlorométhane/trifluorométhyl-3-phénol ; 4/1 : s > 8 g/100cm³
. dichlorométhane/chloro-2-phénol ; 4/1 : s > 8 g/100cm³
. dichlorométhane/pentafluoro-2,3,4,5,6-phénol ; 4/1 : s > 8 g/100cm³
. acétate d'éthyle/phénol ; 9/1 : s = 8 g/100cm³

### Exemple 10 : Ester méthylique de la N-tertiobutyloxycarbonyl-pentaglycine (Boc-Gly-Gly-Gly-Gly-Gly-OCH3): ( Boc-gly₅-OCH₃)

La solubilité dans le dichlorométhane a été mesurée par HPLC, avec étalonnage externe, sur une solution saturée de Boc-Gly₅-OCH₃.

Les autres solubilités ont été mesurées comme dans l'exemple 1.
. dichlorométhane : s = 0,33 g/100cm³
. dichlorométhane/phénol ; 20/1 : s = 0,5 g/100cm³
. dichlorométhane/phénol ; 4/1 : s = 4 g/100cm³
. anisole : s < 0,05 g/100cm³
. anisole/phénol ; 4/1 : s = 4 g/100cm³
. chloro-2-phénol : s > 8 g/100cm³
. dichlorométhane/chloro-2-phénol ; 4/1 : 2 > s > 1 g/100cm³
. dichlorométhane/méthoxy-4-phénol; 4/1 : 4 > s > 2 g/100cm³

### Exemple 11 : Ester benzylique de la N-tertiobutyloxycarbonyl-pentaglycine (Boc-Gly-Gly-Gly-Gly-Gly-O-CH₂-Ph)

La solubilité dans le dichlorométhane a été mesurée par HPLC par étalonnage externe sur le surnageant d'une solution saturée ; les autres solubilités ont été mesurées comme dans l'exemple 1 :
. dichlorométhane : s = 0,07 g/100cm³
. dichlorométhane phénol ; 9/1 : s > 5 g/100cm³

A titre comparatif des additifs ne faisant pas partie de l'invention, ont été essayés :
. dichlorométhane/trifluoroéthanol ; 9/1 : s < 2 g/100cm³ *
. dichlorométhane/octanol-2 ; 9/1 : s < 2 g/100cm³ *
. dichlorométhane/acide pivalique ; 9/1 : s < 2 g/100cm³ *
. dichlorométhane/diméthylformamide ; 9/1 : s < 2 g/100cm³ *
. dichlorométhane saturé par LiCl : s < 2 g/100cm³ *

* Les concentrations plus faibles que 2g/100cm³ n'ont pas été essayées.

### Exemple 12 : Ester méthylique de l'acide N-benzyloxy-carbonyl-β benzyl-L-aspartyl-amino-1-cyclopropane-carboxylique (Z-Asp-(OBzl)- Acc-OCH₃)

Solubilités mesurées comme dans l'exemple 1 :
. oxyde de méthyle et de tert. butyle : s < 0,5 g/100cm³
. oxyde de méthyle et de tert. butyle/tert.butyl-4-phénol ; 6/1 : s > 1,2 g/100cm³
. chlorobenzène : s < 1,5 g/100cm³
. chlorobenzène/tert.butyl-4-phénol; 4/1 : s > 20 g/100cm³
. anisole : s > 2,5 g/100cm³
. anisole/tert.butyl-4-phénol; 4/1 : s > 25 g/100cm³
. acétate d'éthyle : s = 2,5 g/100cm³
. acétate d'éthyle/hydroxy-2 pyridine (solution saturée) : 10 > s > 5g/100cm³

### SYNTHESE DE PEPTIDES

### Exemple 13 : Ester propylique de l'acide L-aspartyl-amino-1-cyclopropane-carboxylique (Asp-Acc-Opr).

Remarque: ce dipeptide ester est un édulcorant présentant un pouvoir sucrant environ 200 fois supérieur à celui du saccharose selon C. MAPELLI, M. GARY NEWTON, C.E. RINGOLD et C.H. STAMMER, Int. J.Peptide Protein Res. 30, 498-510 (1987).

### Etape 1 : Condensation à la concentration de 0,66 mole/litre de CH₂ Cl₂ (soit 320 g).

Dans un réacteur tricol en pyrex de 100 cm³, refroidi par un bain d'eau à 17°C et inerté par un courant d'azote sec, sont chargés successivement, sur un pied d'anisole (37,5 cm³) agité, les solides suivants:

L'acide N-benzyloxycarbonyle-β benzyl-aspartique (25 mmoles),
le chlorhydrate de N-éthyl-N′-(diméthylamino-3 propyl) carbodiimide (30 mmoles) et l'hydroxypyridine (42,5 mmoles).
La suspension se fluidifie rapidement et conduit à une solution à laquelle sont ajoutés tour à tour au bout de 15 minutes:

L'amino-1-cyclopropane carboxylate de propyle (30 mmoles) et le tertiobutyl-4-phénol (5 g).

Après une nuit sous agitation, le mélange réactionnel est lavé à 30°C par 25 cm³ d'acide chlorhydrique 2N puis à 3 reprises par 13 cm³ d'une solution aqueuse constituée de 10 cm³ d'eau déminéralisée et de 3 cm³ de saumure saturée en NaCl. Les phases aqueuses réunies sont contre-extraites par 2,5 cm³ d'anisole.

### Etape 2: Hydrogénolyse des groupes protecteurs enchaînée avec l'étape de condensation.

La phase organique (40 cm3) est transférée dans un réacteur en pyrex tricol de 200 cm³ contenant 100 cm³ d'eau distillée et 2,8 g de palladium à 3% sur carbone, préalablement inerté sous azote.

Le mélange porté à 55°C est placé à pression ambiante sous courant d'hydrogène durant 4 heures.

### Etape 3 : Isolement du peptide

Le mélange réactionnel est filtré sur millipore 5 A, le catalyseur est lavé sur le filtre par 10 cm³ d'eau à 35°C à 2 reprises.
La phase organique est décantée et écartée.

La phase aqueuse est lavée par 30 cm³ d'acétate d'éthyle à 3 reprises puis concentrée par distillation sous vide jusqu'à une masse résiduelle de 60 g.
Il se produit spontanément une cristallisation.

La suspension, additionnée de 240 cm³ d'acétone est filtrée sur verre frité de porosite n° 4.
Le solide lavé à l'acétone et séché à poids constant pèse 4,41 g.
Le filtrat concentré à sec est dissous dans 11 cm³ d'eau.
L'addition d'acétone (66 cm³) permet d'isoler un second lot de solide pesant après séchage sous vide 1,52 g.

### Résultats :

L'ester propylique de l'acide L-aspartyl-amino-1-cycloprane-carboxylique monohydraté, ainsi obtenu avec un rendement de 86 %, est un solide blanc fondant à 179°C.

Son spectre RMN ¹H enregistré à 360 MHz en solution dans le DMSO est conforme.
Analyse élémentaire : C 11, H 18, N 2, 05, 1H₂O
Calculé : C 47,83 ; H 7,3 ; N 10,14 ; 0 34,76
Trouvé : C 47,69 ; H 7,1 ; N 10,01 ; 0 34,81
Teneur en eau (Karl Fischer) : 6,48 %. Dosage potentiométrique
   Fonction amine : 102 %
   Fonction acide : 98 %

### Exemple 14

A titre de comparaison un essai selon l'exemple 13 dans lequel on omet soit l'addition de tertiobutyl-4 phénol, soit l'addition d'hydroxy-2 pyridine conduit rapidement à un mélange réactionnel inagitable.

### PURIFICATION DE PEPTIDES PAR EXTRACTION LIQUIDE-LIQUIDE

### Exemple 15: Ester benzylique de la glycyl-glycyl-L-phénylalanyl-L-leucine (Gly-Gly-Phe-Leu-OBzl)

En vue d'une purification de ce tétrapeptide, par lavage à l'eau à l'issue de sa synthèse, la partition de ce peptide entre le dichlorométhane et l'eau a été étudiée comme suit. Une solution de chlorhydrate de Gly-Gly-Phe-Leu-OBzl (0,9 mmole) dans le

dichlorométhane (12 cm³) est agitée fortement en présence d'une solution aqueuse de KHCO₃ (2 N; 3 cm³). Ce mélange fournit une émulsion qui reste stable durant plusieurs jours. Un essai comparatif a été réalisé en présence de phénol:

### Exemple 16

Un essai de partition réalisé selon l'exemple 15 après addition de 3,9 g de phénol à la solution organique, fournit après une agitation identique deux phases liquides limpides, dont la décantation est totale en moins de 15 minutes.

L'analyse HPLC montre que la phase aqueuse renferme seulement 0,4% du tétrapeptide engagé.

### Exemple 17: Ester benzylique de la N-tertiobutyloxycarbonyl-L-phénylalanyl-L-valine (Boc-Phe-Val-OBzl).

Dans un réacteur en verre pyrex de 50 cm³ sont introduits successivement à température ambiante:
l'ester hydroxy-2 pyridinique de la N-tert.butyloxycarbonyl-L-phénylalanine (3,6 mmoles ; 1,23 g)
Le chlorhydrate de l'ester benzylique de la Valine (3 mmoles ; 0,73g)

Le dichlorométhane (15 cm³) puis sous agitation la N-méthyl morpholine (3 mmoles).

Après 68 heures à température ambiante le mélange réactionnel est lavé successivement par 5 cm³ d'acide sulfurique dilué (95p pH2), par 5 cm³ d'eau, puis par 5 cm³ de KHCO₃. 2N en solution dans l'eau.
Il se forme alors une émulsion stable.

Au contraire, lorsque l'on emploie un mélange dichlorométhane/phénol (9/1) comme solvant de réaction, tous les lavages se déroulent sans difficulté et les décantations sont rapides.

### EXEMPLE 18

Sujet : Etude de l'influence du phénol au cours d'un couplage peptidique.

Couplage étudié:
Boc-Phe-O-Py + HCl, Val-OBzl + NMM
CH₂ Cl₂ (avec ou sans phénol)
Boc-Phe-Val OBzl + HCl, NMM

### Mode opératoire

Le couplage a été réalisé en mettant en contact dans du chlorure de méthylène à reflux, d'une part 1,2 équivalent d'ester hydroxy 2 pyridinique de N ter butyloxy L phénylalanine (Boc-Phe-OPy) et d'autre part un équivalent de chlorhydrate d'ester benzylique de la valine (Val OBzl) un équivalent en présence de N méthyl morpholine (NMM).

On a mesuré la disparition de Val OBzl et l'apparition dudit peptide. La corrélation est parfaite aux indéterminations de mesures près.

Les résultats sont rassemblés dans le tableau suivant, dans lequel on fait apparaître le rendement de peptide et, ce qui est beaucoup plus significatif des problèmes d'activation et de cinétique, le Val OBzl résiduel.
Deux types d'essais ont été réalisés en milieu parfaitement homogène, d'une part avec du phénol C6 H50H dans du chlorure de méthylène (10 % en masse de phénol), et d'autre part sans phénol.

Les résultats montrent une différence de cinétique extrêmement significative, puisqu'on obtient 90 % du dit peptide en 2 heures dans le chlorure de méthylène seul, et en 20 minutes dans le mélange chlorure de méthylène phénol 10 % en masse.

### RESULTATS :

Dans le tableau est précisé le rendement de couplage ainsi que le pourcentage de Val OBzl au cours du temps.

| | TEMPS | 10 mn | 30 mn | 1 heure | 2 h 30 |
|---|---|---|---|---|---|
| Rendement de Couplage | sans φOH | 56 % | 80 % | 86 % | 95 % |
| | avec φOH | 78 % | 93 % | 96 % | 99 % |
| Val OBzl Résiduel | sans φOH | 44 % | 20 % | 14 % | 5 % |
| | avec φOH | 22 % | 7 % | 4 % | 1 % |

### Exemple 19

**Influence de l'ortho-crésol sur la cinétique d'un couplage peptidique.**

### REACTION :

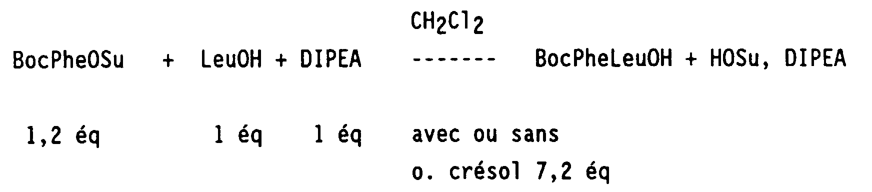

### Mode opératoire

Le couplage a été réalisé en mettant en contact dans le chlorure de méthylène à température ambiante, 1,2 équivalent d'ester d'hydroxysuccinimide de N-tert-butyloxycarbonyl-L-phénylalanine (BocPheOSU) et 1 équivalent de leucine libre (LeuOH) en présence de 1 équivalent de diisopropyléthylamine (DIPEA).
Deux types d'essais ont été réalisés en milieu hétérogène (la leucine est insoluble), d'une part avec 7,2 équivalents d'orthocrésol et d'autre part sans ortho-crésol.

On a mesuré l'apparition du dipeptide formé BocPheLeuOH.

| SOLVANT | Rendement de BocPheLeuOH/Leucine | |
|---|---|---|
| | 1 h | 2 h |
| CH₂Cl₂ | 9 % | 17 % |
| CH₂Cl₂/o.crésol | 57 % | 72 % |

Les résultats montrent une influence importante de l'orthocrésol sur la cinétique de couplage. On multiplie par six la formation de dipeptide en 1 heure de réaction en présence d'ortho-crésol.

## Revendications

1. Milieu réactionnel et solubilisant utile pour la synthèse et/ou la purification peptidique caractérisé par le fait qu'il comporte un diluant A choisi dans le groupe des solvants non miscibles à l'eau et un phénol B, et le rapport massique entre ledit phénol B et ledit diluant A est compris entre 1/200 et 1, de préférence compris entre 1/20 et 1/2 et qu'il comporte au moins un acide aminé ou au moins un peptide à une concentration au moins égale à 10⁻² M.

2. Milieu selon la revendication 1, caractérisé par le fait que ledit au moins un acide aminé ou au moins un peptide est présent à une concentration au moins égale à 0,05M

3. Milieu selon l'une des revendications 1 et 2, caractérisé par le fait que ledit diluant est choisi dans le groupe constitué par les aromatiques, les éthers, les esters et les solvants halogénés.

4. Milieu selon l'une des revendications 1 à 3 caractérisé par le fait que ledit phénol est choisi dans le groupe des composés répondant à la formule générale suivante I :
(R₁)n - Aᵣ - O - H (I)
dans laquelle :
- Aᵣ représente un radical aromatique monocyclique, polycyclique, hétérocyclique ou non ;
- Les substituants R₁, semblables ou différents, représentent:
- un motif halogène, de préférence fluor, chlore, Brome,
- un groupe -Z=R₂, où Z peut être,
- une simple liaison;
- un atome d'oxygène;
où R₂ représente un atome d'hydrogène, un radical alcoyle ou aryle éventuellement hydroxylés ou mono- ou poly halogénés d'au plus 8 atomes de carbone,
- n représente le nombre de substituants et est égal à 0 ou à un entier au plus égal au nombre de positions substituables sur les noyaux aromatiques et de leurs mélanges ;

5. Milieu selon l'une des revendications 1 à 4 que ledit phénol B a un nombre d'atomes de carbone d'au plus 30 atomes de carbone de préférence au plus 20 atomes de carbone.

6. Milieu selon l'une des revendications 1 à 5 caractérisé par le fait que les positions vicinales de la fonction phénol sont non substituées ou occupés par des groupes non encombrants.

7. Milieu selon l'une des revendications 4 à 6 caractérisé par le fait que le radical -Aᵣ est un aromatique monocyclique ayant de préférence 6 chaînons.

8. Milieu selon l'une des revendications 4 à 7 caractérisé par le fait que dans la formule I, les radicaux R sont choisis parmi le groupe constitué par :
- les radicaux méthyle, éthyle, propyles, butyles,
- les radicaux trifluorométhyle et pentafluoroéthyle,
- les radicaux méthoxyle, éthoxyle, propyloxyles, butyloxyles,
- phényle, hydroxy phényle, et Aᵣ OH,
- les radicaux phényl oxyles, hydroxy phényloxyles,
- atomes de fluor, de chlore et de Brome.

9. Milieu selon l'une des revendications 4 à 8 caractérisé par le fait que dans la formule I n est au plus égal à 5.

10. Milieu selon l'une des revendications 1 à 6, 8 et 9 caractérisé par le fait que ledit phénol B est choisi dans le groupe constitué par les :
- hydroxy pyridines éventuellement monosubstituées,
- hydroxy quinoléines éventuellement monosubstituées,
- les mono halogéno phénols (de préférence mono chloro),
- les poly halogéno phénols, (de préférence polyfluoro),
- les phénols mono- ou disubstitués par des radicaux alkyles de C₁ à C₄, alcoxyles de C₁ à C₄, des perfluoroalkyles de C₁ à C₂ et le 2,2,2, trifluoro éthyle,
- les diphénols,
- le phénol au sens étroit,
- les naphtols éventuellement mono- ou di- substitués.

11. Milieu selon les revendication 1 à 10 caractérisé par le fait que ledit au moins un acide aminé ou au moins un peptide est un acide aminé C protégé.

12. Milieu selon la revendication 1 à 10, caractérisé par le fait que ledit au moins un acide aminé ou au moins un peptide est un acide aminé N protégé.

13. Milieu selon la revendication 12, caractérisé par le fait que ledit acide aminé est activé sur la fonction acide.

14. Milieu selon les revendications 1 à 10, caractérisé par le fait que ledit au moins un acide aminé ou au moins un peptide est un peptide C protégé.

15. Milieu selon les revendications 1 à 10, caractérisé par le fait que le dit peptide est N protégé.

16. Milieu selon la revendication 15 caractérisé par le fait que ledit peptide est activé sur la fonction acide.

17. Milieu selon l'une des revendications 1 à 10 et 14 à 16, caractérisé par le fait que ledit au moins un acide aminé ou au moins un peptide est un peptides de 2 à 20 acides aminés, avantageusement de 3 à 20.

18. Milieu selon l'une des revendications 1 à 17, caractérisé par le fait qu'il comporte en outre un acide aminé protégé et/ou un peptide protégé.

19. Procédé de synthèse de Peptides éventuellement protégés en milieu liquide, où l'on part d'un peptide ou d'un amino acide initial, protégé sur sa fonction acide et en ce que l'on ajoute un aminoacide activé sur la fonction acide et protégé sur la fonction amine, caractérisé par le fait que les réactifs sont dissous dans un milieu comportant un phénol B et avantageusement un diluant A le rapport massique entre ledit phénol B et ledit diluant A étant alors compris entre 1/200 et 1.

20. Procédé de synthèse de Peptides éventuellement protégés en milieu liquide où l'on part d'un amino acide ou peptide initial, solubilisé selon la revendication 1 et que l'on condense un autre peptide ayant sa fonction acide C terminale activée et sa fonction amine N-terminale protégée, caractérisé par le fait que les réactifs sont dissous dans un milieu comportant un phénol B et avantageusement un diluant A le rapport massique entre ledit phénol B et ledit diluant A étant alors compris entre 1/200 et 1.

21. Procédé selon l'une quelconque des revendications 19 et 20 caractérisé par le fait que la fonction amine est protégée par un groupe carbamate ou par réaction avec un composé bétadicarbonylé.

22. Procédé selon l'une quelconque des revendications 19 à 21, caractérisé en ce que la fonction acide C-Terminale du peptide ou de l'amino acide initial est protégée de préférence par estérification ou par amidation.

23. Procédé selon l'une quelconque des revendications 19 à 21, caractérisé en ce que la fonction acide des réactifs N-protégés est activée par un chlorure d'acide organique ou minéral, par un chloroformiate d'alkyle, par un carbodiimide ou par un ester activé ou par un acyle imidazole.

24. Procédé selon l'une quelconque des revendications 19 à 21, caractérisé en ce qu'après condensation d'un amino acide ou d'un peptide N- protégé avec le peptide ou l'amino acide initial, la fonction amine N-terminale est libérée par hydrogénolyse ou par acidolyse ou par basolyse.

25. Procédé selon l'une quelconque des revendications 19 à 24 caractérisé en ce que l'amino-acide ou le peptide C-terminal protégé initial est introduit dans le milieu réactionnel, puis l'acide aminé ou le peptide activé sur sa fonction acide et protégé sur sa fonction amine est ajouté, l'ordre d'addition des réactifs étant indifférents; puis la fonction amine est libérée et enfin un nouvel acide aminé ou un nouveau peptide activé sur sa fonction acide et protégé sur sa fonction amine, est introduit.

26. Procédé selon l'une quelconque des revendications 19 à 25, caractérisé en ce que il comporte en outre une étape dans laquelle les co-produits et les excès de réactifs sont éliminés de la phase organique par lavage avec des solutions aqueuses acides, basiques ou neutres.

27. Réactif comportant comme composant en vue d'une introduction successive ou simultanée :
a) un diluant A choisi dans le groupe des solvants non miscibles à l'eau
b) un phénol B
c) un oligopeptide de 1 à 50, de préférence de 1 à 20 acides aminés dont ni le C , ni le N terminal n'est protégé, et caractérisé par le fait que le rapport massique entre ledit phénol B et ledit diluant A est compris entre 1/200 et 1 et par le fait que il comporte au moins un acide aminé ou au moins un peptide à une concentration au mois égale à 10⁻²M.

28. utilisation de phénol(s) pour la solubilisation de peptides dans un milieu réactionnel ledit phénol étant choisi dans le groupe des composés répondant à la formule générale suivante I:
(R₁)n - Aᵣ - O - H (I)
dans laquelle :
- Aᵣ représente un radical aromatique monocyclique, polycyclique, hétérocyclique ou non ;
- Les substituants R₁, semblables ou différents, représentent:
- un motif halogène, de préférence fluor, chlore, Brome,
- un groupe -Z-R₂, où Z peut être,
- une simple liaison;
- un atome d'oxygène;
où R₂ représente un atome d'hydrogène, un radical alcoyle ou aryle éventuellement hydroxylés ou mono- ou poly halogénés d'au plus 8 atomes de carbone,
- n représente le nombre de substituants et est égal à 0 ou à un entier au plus égal au nombre de positions substituables sur les noyaux aromatiques et de leurs mélanges.

## Patentansprüche

1. Reaktions- und Solubilisierungsmilieu, verwendbar für die Synthese und/oder die Reinigung von Peptiden, dadurch gekennzeichnet, daß es ein Verdünnungsmittel A, das aus der Gruppe von nicht mit Wasser mischbaren Lösemitteln ausgewählt ist, und ein Phenol B enthält, daß das Massenverhältnis von Phenol B und Verdünnungsmittel A zwischen 1/200 und 1, vorzugsweise zwischen 1/20 und 1/2, liegt und daß das Milieu mindestens eine Aminosäure oder mindestens ein Peptid mit einer Konzentration von mindestens 10⁻² M enthält.

2. Milieu nach Anspruch 1, dadurch gekennzeichnet, daß mindestens eine Aminosäure oder mindestens ein Peptid mit einer Konzentration von mindestens 0,05 M vorhanden ist.

3. Milieu nach einem der Ansprüche 1 und 2, dadurch gekennzeichnet, daß das Verdünnungsmittel aus der Gruppe von Aromaten, Ethern, Estern und halogenierten Lösemitteln ausgewählt ist.

4. Milieu nach einem der Ansprüche 1 bis 3, dadurch gekennzeichnet, daß das Phenol ausgewählt ist aus der Gruppe von Verbindungen, die der folgenden allgemeinen Formel I entsprechen
(R₁)n-Aᵣ-O-H (I)
in der
- Aᵣ einen monocyclischen, polycyclischen, gegebenenfalls heterocyclischen aromatischen Rest darstellt;
- die Substituenten R₁, gleich oder verschieden, darstellen:
- einen Halogenrest, vorzugsweise Fluor, Chlor oder Brom,
- eine Gruppe -Z-R₂, wobei Z
- eine Einfachbindung;
- ein Sauerstoffatom darstellen kann,
wobei R₂ ein Wasserstoffatom oder einen mono- oder polyhalogenierten oder gegebenenfalls hydroxylierten Aryl- oder Alkylrest mit höchstens 8 Kohlenstoffatomen darstellt und
n die Zahl der Substituenten darstellt und gleich 0 oder eine ganze Zahl ist, die höchstens gleich der Zahl der substituierbaren Positionen an den aromatischen Kernen oder ihrer Mischungen ist.

5. Milieu nach einem der Ansprüche 1 bis 4, dadurch gekennzeichnet, daß das Phenol B eine Kohlenstoffatomanzahl von höchstens 30 Kohlenstoffatomen, vorzugsweise von höchstens 20 Kohlenstoffatomen, besitzt.

6. Milieu nach einem der Ansprüche 1 bis 5, dadurch gekennzeichnet, daß die zur Phenolfunktion benachbarten Funktionen nicht substituiert sind oder mit nichtsperrigen Gruppen besetzt sind.

7. Milieu nach einem der Ansprüche 4 bis 6, dadurch gekennzeichnet, daß der Rest Aᵣ ein monocyclischer aromatischer Rest mit vorzugsweise 6 Kettengliedern ist.

8. Milieu nach einem der Ansprüche 4 bis 7, dadurch gekennzeichnet, daß in der Formel I die Reste R ausgewählt sind aus der Gruppe von
- Methyl-, Ethyl-, Propyl- und Butylresten
- Trifluormethyl- und Pentafluorethylresten
- Methoxyl-, Ethoxyl-, Propyloxyl- und Butyloxylresten
- Phenylresten, Hydroxyphenylresten und AᵣOH
- Phenyloxyl- und Hydroxyphenyloxylresten
- Fluor-, Chlor- und Bromatomen.

9. Milieu nach einem der Ansprüche 4 bis 8, dadurch gekennzeichnet, daß in der Formel I n höchstens gleich 5 ist.

10. Milieu nach einem der Ansprüche 1 bis 6, 8 und 9, dadurch gekennzeichnet, daß das Phenol B ausgewählt ist aus der Gruppe von
- gegebenenfalls monosubstituierten Hydroxypyridinen
- gegebenenfalls monosubstituierten Hydroxychinolinen
- Monohalogenphenolen (vorzugsweise Monochlorphenolen)
- Polyhalogenphenolen (vorzugsweise Polyfluorphenolen)
- Phenolen, die mono- oder disubstituiert sind mit C₁- bis C₄-Alkylresten, C₁- bis C₄-Alkoxylresten, C₁- bis C₂-Perfluoralkylresten und 2,2,2-Trifluorethylresten.
- Diphenolen
- Phenol im engeren Sinn
- gegebenenfalls mono- oder disubstituierten Naphtholen.

11. Milieu nach einem der Ansprüche 1 bis 10, dadurch gekennzeichnet, daß die mindestens eine Aminosäure oder das mindestens eine Peptid eine C-geschützte Aminosäure ist.

12. Milieu nach Anspruch 1 bis 10, dadurch gekennzeichnet, daß die mindestens eine Aminosäure oder das mindestens eine Peptid eine N-geschützte Aminosäure ist.

13. Milieu nach Anspruch 12, dadurch gekennzeichnet, daß die Aminosäure an der Säurefunktion aktiviert ist.

14. Milieu nach den Ansprüchen 1 bis 10, dadurch gekennzeichnet, daß die mindestens eine Aminosäure oder das mindestens eine Peptid ein C-geschütztes Peptid ist.

15. Milieu nach den Ansprüchen 1 bis 10, dadurch gekennzeichnet, daß das Peptid N-geschützt ist.

16. Milieu nach Anspruch 15, dadurch gekennzeichnet, daß das Peptid an der Säurefunktion aktiviert ist.

17. Milieu nach einem der Ansprüche 1 bis 10 und 14 bis 16, dadurch gekennzeichnet, daß die mindestens eine Aminosäure oder das mindestens eine Peptid ein Peptid mit 2 bis 20 Aminosäuren, vorzugsweise mit 3 bis 20 Aminosäuren, ist.

18. Milieu nach einem der Ansprüche 1 bis 17, dadurch gekennzeichnet, daß es außerdem eine geschützte Aminosäure und/oder ein geschütztes Peptid enthält.

19. Verfahren zur Synthese von gegebenenfalls geschützten Peptiden in flüssigem Milieu, wobei man von einem anfänglichen Peptid oder einer anfänglichen Aminosäure ausgeht, die das an der Säurefunktion geschützt ist, und wobei man eine Aminosäure hinzugibt, die an der Säurefunktion aktiviert und an der Aminfunktion geschützt ist, dadurch gekennzeichnet, daß die Reagenzien in einem Milieu gelöst werden, das ein Phenol B und vorzugsweise ein Verdünnungsmittel A enthält, wobei das Massenverhältnis zwischen dem Phenol B und dem Verdünnungsmittel A zwischen 1/200 und 1 liegt.

20. Verfahren zur Synthese von gegebenenfalls geschützten Peptiden in flüssigem Milieu, wobei man von einer anfänglichen Aminosäure oder einem anfänglichen Peptid ausgeht, das gemäß Anspruch 1 solubilisiert wird, und wobei man ein weiteres Peptid mit einer aktivierten C-terminalen Säurefunktion und einer geschützten N-terminalen Aminfunktion kondensiert, dadurch gekennzeichnet, daß die Reagenzien in einem Milieu gelöst werden, das ein Phenol B und vorzugsweise ein Verdünnungsmittel A enthält, wobei das Massenverhältnis zwischen dem Phenol B und Verdünnungsmittel A dann zwischen 1/200 und 1 liegt.

21. Verfahren nach einem der Ansprüche 19 und 20, dadurch gekennzeichnet, daß die Aminfunktion durch eine Carbamatgruppe oder durch Reaktion mit einer Betadicarbonylverbindung geschützt ist.

22. Verfahren nach einem der Ansprüche 19 bis 21, dadurch gekennzeichnet, daß die C-terminale Säurefunktion des anfänglich eingesetzten Peptids oder der anfänglich eingesetzten Aminosäure vorzugsweise durch Veresterung oder Amidierung geschützt ist.

23. Verfahren nach einem der Ansprüche 19 bis 21, dadurch gekennzeichnet, daß die Säurefunktion der N-geschützten Reagenzien aktiviert ist durch ein organisches oder mineralisches Säurechlorid, durch ein Alkylchlorformiat, durch ein Carbodiimid, durch einen aktivierten Ester oder durch ein Acylimidazol.

24. Verfahren nach einem der Ansprüche 19 bis 21, dadurch gekennzeichnet, daß nach Kondensation einer N-geschützten Aminosäure oder eines N-geschützten Peptids mit dem anfänglich eingesetzten Peptid oder der anfänglich eingesetzten Aminosäure die N-terminale Aminfunktion durch Hydrogenolyse, durch Acidolyse oder durch Basolyse freigesetzt wird.

25. Verfahren nach einem der Ansprüche 19 bis 24, dadurch gekennzeichnet, daß die anfänglich eingesetzte, geschützte C-terminale Aminosäure oder das anfänglich eingesetzte, geschützte C-terminale Peptid in das Reaktionsmilieu eingebracht wird, dann die an ihrer Säurefunktion aktivierte und an ihrer Aminfunktion geschützte Aminosäure oder das an seiner Säurefunktion aktivierte und an seiner Aminfunktion geschützte Peptid hinzugegeben wird, wobei die Reihenfolge der Zugabe der Reagenzien gleichgültig ist; dann die Aminfunktion freigesetzt wird und schließlich eine neue, an ihrer Säurefunktion aktivierte und an ihrer Aminfunktion geschützte Aminosäure oder ein neues, an seiner Säurefunktion aktiviertes und an seiner Aminfunktion geschütztes Peptid hinzugegeben wird.

26. Verfahren nach einem der Ansprüche 19 bis 25, dadurch gekennzeichnet, daß es außerdem einen Schritt umfaßt, bei dem die Nebenprodukte und die Reagenzienüberschüsse aus der organischen Phase durch Waschen mit sauren, basischen oder neutralen wäßrigen Lösungen entfernt werden.

27. Reagenz, enthaltend als Bestandteil im Hinblick auf eine sukzessive oder gleichzeitige Zugabe:
a) ein Verdünnungsmittel A, ausgewählt aus der Gruppe von nicht mit Wasser mischbaren Lösemitteln
b) ein Phenol B
c) ein Oligopeptid mit 1 bis 50, vorzugsweise mit 1 bis 20 Aminosäuren, die weder am endständigen C noch am endständigen N geschützt sind und das dadurch gekennzeichnet ist, daß das Massenverhältnis zwischen dem Phenol B und dem Verdünnungsmittel A zwischen 1/200 und 1 liegt und daß es mindestens eine Aminosäure oder mindestens ein Peptid mit einer Konzentration von mindestens 10⁻² M enthält.

28. Verwendung von Phenol(en) zur Solubilisierung von Peptiden in einem Reaktionsmilieu, wobei das Phenol ausgewählt ist aus der Gruppe von Verbindungen, die der folgenden allgemeinen Formel I entsprechen
(R₁)n-Aᵣ-O-H (I)
in der
- Aᵣ einen monocyclischen, polycyclischen, gegebenenfalls heterocyclischen aromatischen Rest darstellt;
- die Substituenten R₁, gleich oder verschieden, darstellen:
- einen Halogenrest, vorzugsweise Fluor, Chlor oder Brom,
- eine Gruppe -Z-R₂, wobei Z
- eine einfache Bindung;
- ein Sauerstoffatom darstellen kann,
wobei R₂ ein Wasserstoffatom oder einen gegebenenfalls hydroxylierten oder mono- oder polyhalogenierten Alkyl- oder Arylrest mit höchstens 8 Kohlenstoffatomen darstellt,
- und n die Zahl der Substituenten darstellt und gleich 0 oder eine ganze Zahl ist, die höchstens gleich der Zahl der substituierbaren Positionen an den aromatischen Kernen oder ihrer Mischungen ist.

## Claims

1. Reaction and dissolving medium for peptide synthesis and/or purification, characterized in that it comprises a diluent A chosen from the group of water-immiscible solvents and a phenol B, in that the ratio by mass of the said phenol B to the said diluent A is between 1/200 and 1, preferably between 1/20 and 1/2, and in that it comprises at least one amino acid or at least one peptide at a concentration at least equal to 10⁻²M.

2. Medium according to Claim 1, characterized in that the said at least one amino acid or at least one peptide is present at a concentration at least equal to 0.05M.

3. Medium according to one of Claims 1 and 2, characterized in that the said diluent is chosen from the group comprising aromatic compounds, ethers, esters and halogenated solvents.

4. Medium according to one of Claims 1 to 3, characterized in that the said phenol is chosen from the group of compounds corresponding to the following general formula I:
(R₁)n-Aᵣ-O-H (I)
in which:
- Aᵣ represents a monocyclic or polycyclic aromatic radical which may or may not be heterocyclic;
- the substituents R₁, which are similar or different, represent:
- a halogen unit, preferably fluorine, chlorine or bromine,
- a group -Z-R₂, where Z can be,
- a single bond;
- an oxygen atom;
where R₂ represents a hydrogen atom or an alkyl or aryl radical which may be hydroxylated or mono- or polyhalogenated and has at most 8 carbon atoms,
- and n represents the number of substituents and is 0 or an integer at most equal to the number of positions available for substitution on the aromatic nuclei and their mixtures.

5. Medium according to one of Claims 1 to 4, characterized in that the said phenol B has a number of carbon atoms of at most 30 carbon atoms, preferably at most 20 carbon atoms.

6. Medium according to one of Claims 1 to 5, characterized in that the vicinal positions of the phenol function are unsubstituted or occupied by non-obstructing groups.

7. Medium according to one of Claims 4 to 6, characterized in that the radical -Aᵣ is a monocyclic aromatic compound preferably having 6 members.

8. Medium according to one of Claims 4 to 7, characterized in that, in the formula I, the radicals R are chosen from the group comprising:
- the methyl, ethyl, propyl and butyl radicals,
- the trifluoromethyl and pentafluoroethyl radicals,
- the methoxy, ethoxy, propoxy and butoxy radicals,
- phenyl, hydroxyphenyl and AᵣOH,
- the phenoxy and hydroxyphenoxy radicals, and
- fluorine, chlorine and bromine atoms.

9. Medium according to one of Claims 4 to 8, characterized in that n in the formula I is at most 5.

10. Medium according to one of Claims 1 to 6, 8 and 9, characterized in that the said phenol B is chosen from the group comprising:
- hydroxypyridines, which may be monosubstituted,
- hydroxyquinolines, which may be monosubstituted,
- monohalogenophenols (preferably monochlorophenols),
- polyhalogenophenols (preferably polyfluorophenols),
- phenols monosubstituted or disubstituted by C₁ to C₄ alkyl radicals, C₁ to C₄ alkoxy radicals, C₁ to C₂ perfluoroalkyl radicals and 2,2,2-trifluoroethyl,
- diphenols,
- phenol in the narrow sense, and
- naphthols, which may be monosubstituted or disubstituted.

11. Medium according to Claim 1 to 10, characterized in that the said at least one amino acid or at least one peptide is a C-protected amino acid.

12. Medium according to Claim 1 to 10, characterized in that the said at least one amino acid or at least one peptide is an N-protected amino acid.

13. Medium according to Claim 12, characterized in that the said amino acid is activated on the acid function.

14. Medium according to Claims 1 to 10, characterized in that the said at least one amino acid of at least one peptide is a C-protected peptide.

15. Medium according to Claims 1 to 10, characterized in that the said peptide is N-protected.

16. Medium according to Claim 15, characterized in that the said peptide is activated on the acid function.

17. Medium according to one of Claims 1 to 10 and 14 to 16, characterized in that the said at least one amino acid or at least one peptide is a peptide having from 2 to 20, advantageously from 3 to 20, amino acids.

18. Medium according to one of Claims 1 to 17, characterized in that it additionally comprises a protected amino acid and/or a protected peptide.

19. Method for the synthesis of peptides, which may be protected, in a liquid medium, where the starting material is an initial peptide or amino acid protected on its acid function and where an amino acid activated on the acid function and protected on the amine function is added, characterized in that the reagents are dissolved in a medium comprising a phenol B and advantageously a diluent A, the ratio by mass of the said phenol B to the said diluent A then being between 1/200 and 1.

20. Method for the synthesis of peptides, which may be protected, in a liquid medium, where the starting material is an initial amino acid or peptide, dissolved according to Claim 1, and where a condensation reaction is carried out with another peptide having its C-terminal acid function activated and its N-terminal amine function protected, characterized in that the reagents are dissolved in a medium comprising a phenol B and advantageously a diluent A, the ratio by mass of the said phenol B to the said diluent A then being between 1/200 and 1.

21. Method according to either one of Claims 19 and 20, characterized in that the amine function is protected by a carbamate group or by reaction with a β-dicarbonyl compound.

22. Method according to any one of Claims 19 to 21, characterized in that the C-terminal acid function of the initial peptide or amino acid is protected, preferably by esterification or by amidation.

23. Method according to any one of Claims 19 to 21, characterized in that the acid function of the N-protected reagents is activated by an organic or inorganic acid chloride, by an alkyl chloroformate, by a carbodiimide or by an activated ester or by an acylimidazole.

24. Method according to any one of Claims 19 to 21, characterized in that, after the condensation reaction of an N-protected amino acid or peptide with the initial peptide or amino acid, the N-terminal amine function is liberated by hydrogenolysis or by acidolysis or by basolysis.

25. Method according to any one of Claims 19 to 24, characterized in that the initial C-terminal protected amino acid or peptide is introduced into the reaction medium, the amino acid or peptide activated on its acid function and protected on its amine function is then added, the order in which the reagents are added being arbitrary; the amine function is then liberated and, finally, a new amino acid or a new peptide activated on its acid function and protected on its amine function is introduced.

26. Method according to any one of Claims 19 to 25, characterized in that it additionally includes a step in which the co-products and the excess reagents are removed from the organic phase by washing with acid, basic or neutral aqueous solutions.

27. Reagent comprising as component with a view to successive or simultaneous introduction:
a) a diluent A chosen from the group of water-immiscible solvents
b) a phenol B
c) an oligopeptide having from 1 to 50, preferably from 1 to 20, amino acids in which neither the C-terminal nor the N-terminal is protected, and characterized in that the ratio by mass of the said phenol B to the said diluent A is between 1/200 and 1, and in that it comprises at least one amino acid or at least one peptide at a concentration at least equal to 10⁻²M.

28. Use of phenol(s) for dissolving peptides in a reaction medium, the said phenol being chosen from the group of compounds corresponding to the following general formula I:
(R₁)n-Aᵣ-O-H (I)
in which:
- Aᵣ represents a monocyclic or polycyclic aromatic radical which may or may not be heterocyclic;
- the substituents R₁, which are similar or different, represent:
- a halogen unit, preferably fluorine, chlorine or bromine,
- a group -Z-R₂, where Z can be,
- a single bond;
- an oxygen atom;
where R₂ represents a hydrogen atom or an alkyl or aryl radical which may be hydroxylated or mono- or polyhalogenated and has at most 8 carbon atoms,
- and n represents the number of substituents and is 0 or an integer at most equal to the number of positions available for substitution on the aromatic nuclei and their mixtures.
